# EUROPÄISCHE PATENTSCHRIFT

(11) **EP 2 032 052 B1**
(45) Veröffentlichungstag und Bekanntmachung des Hinweises auf die Patenterteilung: **31.12.2014**
(21) Anmeldenummer: 07765573.6
(22) Anmeldetag: 22.06.2007
(51) Int. Cl.: A61B 17/34, A61M 16/04

(54) **VORRICHTUNG ZUM EINBRINGEN EINER TRACHEALKANÜLE IN EIN TRACHEOSTOMA**
DEVICE FOR INSERTING A TRACHEAL CANNULA INTO A TRACHEOSTOMA
DISPOSITIF PERMETTANT L'INSERTION D'UNE CANULE TRACHÉALE DANS UN TRACHÉOSTOME

(30) Priorität: 26.06.2006 DE 102006029599
(43) Veröffentlichungstag der Anmeldung: 11.03.2009
(73) Patentinhaber: Tracoe Medical Gmbh, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2007/056262
(87) Internationale Veröffentlichungsnummer: WO 2008/000701

(56) Entgegenhaltungen:
- US-A- 4 637 388
- US-A- 4 978 334

## Beschreibung

Die vorliegende Erfindung betrifft eine Vorrichtung mit einem Führungskatheter zum Einbringen einer Trachealkanüle in ein Tracheostoma mit einem Führungskatheter. Eine derartige Einführhilfe mit Führungskatheter ist aus der EP 0 407 663 B1 bekannt.

Die Methode der Tracheotomie findet sowohl in der Notfalimedizin als auch bei der Langzeitbeatmung Anwendung. Als Alternative zu der klassischen operativen Technik wurden innerhalb der letzten Jahrzehnte auch verschiedene perkutane Tracheotomietechniken entwickelt, insbesondere die perkutane Dilatationstracheotomie.

Bei der perkutanen Dilatationstracheotomie nach Ciaglia bzw. Griggs oder Frova wird über eine Punktionsnadel zunächst ein Führungsdraht in die Luftröhre eingebracht. Nach Dilatation mit einem 14 French 4,67 mm Dilatator kann der Führungsdraht durch Überziehen eines Führungskatheters verstärkt werden. Die Einheit aus Führungsdraht und Führungskatheter dient dann als Leitschiene zur Dilatation des trachealen Punktionskanals mit Hilfe eines gebogenen konischen Dilatators, einer Zange oder eines schraubenförmigen Dilatators. Nach entsprechendem Aufweiten des Punktionskanals und anschließendem Entfernen des Dilatators kann die Tracheotomiekanüle in analoger Weise unter Verwendung von Führungsdraht und Führungskatheter als Leitschiene mit Hilfe einer dafür vorgesehenen Efnführhüfe eingesetzt werden. Beim Kanülenwechseln kann analog vorgegangen werden, wobei auf einen Führungsdraht verzichtet werden kann.

Das Einsetzen der Tracheostomiekanüle nach dem Entfernen des Dilatators sollte dabei sehr schnell erfolgen, da der Patient während dieser Zeit in der Regel nicht beatmet wird. Außerdem besteht das Risiko einer Blutung, da der Druck auf das aufgeweitete Stoma während der Entnahme des Dilatators reduziert wird. Ein weiteres Problem besteht darin, daß sich das Stoma nach erfolgter Dilatation wieder zusammenzieht. Um dies zu kompensieren wird das Stoma bei der Dilatation meist deutlich weiter aufgedehnt als es der Außendurchmesser der Trachealkanüle erfordern würde. Durch diese starke Dilatation steigt das Risiko von Trachealspangenfrakturen.

Um die Tracheotomiekanüle in das bei der perkutanen Dilatationstracheotomie sehr eng angelegte Tracheostoma einführen zu können, verwendet man eine Einführhilfe, deren maximaler Außendurchmesser geringfügig kleiner ist als der Innendurchmesser der Kanüle und deren Spitze ähnlich wie bei den Dilatatoren am distalen Ende (Patientenende) konisch verjüngt ist und die vom proximalen Ende (Arztende) her in die Kanüle eingeführt wird. In diesem Fall gibt es jedoch einen stufenförmigen Kalibersprung am Übergang zwischen der aus dem distalen Ende der Kanüle herausstehenden Einführhilfe und dem Außendurchmesser an der Stirnseite der Kanülenwand. Bei Kanülen mit dicken Wänden kann dieser abrupte Übergang ein ernsthaftes Problem darstellen. Bei dem Versuch die Kanüle einzusetzen, bleibt die Stirnwand der Kanüle häufig an einer der Trachealspangen hängen. Dabei kann es zu einer Fraktur von Knorpelspangen bzw. zu einer Verletzung der Trachearückwand kommen. Einige Hersteller von Tracheotomiekanülen haben versucht, dieses Problem zu lösen, indem sie die Stirnwand der Kanüle konisch nach innen verjüngt haben. Eine Einführhilfe mit einem Führungskatheter, die eine sich am Patientende konisch verjüngende Spitze aufweist und die vom Arztende in die Tracheotomiekanüle eingeführt wird, mit einer Tracheotomiekanüle mit einer solchen konischen Verjüngung der Stirnwand und zusätzlich mit einer geneigt zur Achse verlaufenden Abschrägung des Kanülenendes ist auch im Falle der eingangs genannten EP 0 407 663 B1 vorgesehen. Dies erleichtert zwar die Einführung, es entstehen jedoch scharfe Kanten an der Stirnseite der Kanüle, die beim Tragen der Kanülen Probleme bereiten können.

In der noch nicht veröffentlichten DE 10 2005 021 470 wird zur Lösung dieses Problems eine Einführhilfe beschrieben, mit einer konischen Spitze, die in einem Zustand die distale Stirnseite der Trachealkanüle mindestens teilweise verdeckt, in einem anderen Zustand aber einen kleineren Basisdurchmesser hat, so daß die Einführhilfe in diesem Zustand durch die Trachealkanüle hindurch zurückgezogen werden kann. Diese Erfindung hat sich für Kanülen mit einer dünnen Wandstärke (0,8 mm) und Innendurchmesser der Tracheotomiekanüle von mindestens 8 mm als sehr erfolgreich erwiesen. Die Wandstärke der meisten Kanülen liegt jedoch deutlich oberhalb von 0,8 mm und um diesen Kalibersprung auszugleichen, benötigt man Einführhilfen mit einer konischen Spitze mit entsprechend hoher Wandstärke. Diese lassen sich jedoch aus sterischen Gründen nur noch schwer durch die Tracheotomiekanüle in Richtung Kanülenspitze schieben. Je weicher das Kanülenrohr ist, desto problematischer wird es, die Einführhilfe mit der konischen Spitze durch das Kanülenrohr in Richtung des distalen Endes zu schieben, da sich die konische Spitze leicht im Rohr verkeilt. Auch das Zurückziehen wird dadurch problematischer.

Aus der US 4,637,388 ist eine Einführhilfe für Endotrachealtuben bekannt, die an einem Führungsstab aus ummantelten Aluminium eine konische Spitze aufweist, die nach hinten in einem dünnwandigen Schirm ausläuft, dessen proximales Ende einen größeren Durchmesser hat als der Durchmesser des Tubus und der mit dem distalen Ende des Tubus in Eingriff treten und dieses abdecken soll. Beim Zurückziehen des Führungsstabes klappt der Schirm um und lässt sich so durch die Kanüle zurückziehen. Der Tubus ist so gestaltet, dass er einfach durch die Öffnung zwischen den Stimmbändern geführt werden kann.

Aus der DE 33 22 001 ist ein chirurgisches Instrumentenbesteck zur Herstellung eines Shunts zum Einbringen einer Kehlkopfprotese bekannt. Ein Prothesen-Platzhalter hat die Form eines Röhrchens mit einem außen angesetzten Schirm als Befestigungsteil, der beim Hindurchschieben durch ein entsprechendes Stoma auf der Rückseite das Röhrchen sichern soll.

Aus der US 4,502,482 ist wiederum eine Einführhilfe für Endotrachealtuben bekannt. Der Tubus weist am distalen Ende eine stumpfe, atraumatische Form auf. Eine innere Einführhilfe in dem Tubus hat ein abgeschrägtes Ende, um das Einbringen des Tubus durch den Kehlkopf zu erleichtern. Der Tubus weist an seinem distalen Ende Vorsprünge auf, die Einkerbungen bilden, wobei die Einführhilfe entsprechende Vorsprünge aufweist, die mit den Einkerbungen am Tubus in Eingriff treten. Bei einer Ausführungsform ist eine geschlitzte konische Spitze vorgesehen, die kollabierbar ist.

Ein weiteres Problem besteht darin, daß das Verhältnis zwischen freier Querschnittsfläche und Wandstärke der konischen Spitze sehr ungünstig wird, wenn der Innendurchmesser einer Tracheotomiekanüle weniger als 8 mm beträgt, und die konische Spitze dadurch nur sehr schwer in den Zustand mit kleinem Basisdurchmesser gebracht werden kann.

Bei bekannten Trachealkanülen mit Einführhilfen besteht zusätzlich das Problem, daß die Einführhilfe nach dem Einsetzen der Trachealkanüle in die Trachea des Patienten nur schwer rückziehbar ist. Dies ist insbesondere dann der Fall, wenn sehr wenig Spiel zwischen der Einführhilfe und der Trachealkanüle vorhanden ist. Auch bei handelsüblichen weichen Kanülen aus Silikon oder PVC und bei Kanülen, deren Biegung so ausgeführt ist, daß die Einführhilfe während des Herausziehens deformiert werden muß, ist das Entfernen der Einführhilfe nur mit erheblichem Kraftaufwand möglich. Dieses Problem kann zwar durch die Verwendung von Gleitmitteln reduziert werden, es besteht jedoch das Risiko, daß der behandelnde Arzt diesen Arbeitsschritt versehentlich überspringt.

Ausgehend von diesem Stand der Technik liegt der vorliegenden Erfindung die Aufgabe zugrunde, eine Vorrichtung zum Einbringen einer Trachealkanüle in ein Tracheostoma zur Verfügung zu stellen, die eine Verletzung durch die distale stirnseitige Fläche der Kanüle verhindert und die geeignet ist für die Anwendung bei sehr flexiblen Kanülen, bei Kanülen mit engem Innendurchmesser und/oder mit großer Wandstärke. Vorzugsweise soll eine Vorrichtung zu Verfügung gestellt werden, mit der auch die Dilatation des Stomas und das Einbringen der Trachealkanüle in einem Schritt durchgeführt werden kann.

Diese Aufgabe wird dadurch gelöst, daß der Führungskatheter einen Schirm aufweist, der in der Nähe des distalen Endes des Führungskatheters an diesem angebracht ist und der aus einem flexiblen Material besteht und im wesentlichen die Form einer distal gerichteten konischen Spitze hat, wobei das Rohr des Führungskatheters durch die Achse dieser Spitze verläuft, mit einem Basisaußendurchmesser, der in einem ersten Zustand in etwa dem Außendurchmesser der Trachealkanüle entspricht und in einem zweiten Zustand durch Verformung kleiner ist als der Innendurchmesser der Trachealkanüle und damit durch die Trachealkanüle rückziehbar ist,_wobei ein hohlzylinderförmiger Verdrängungskörper, dessen Außendurchmesser kleiner ist als der Innendurchmesser der Kanüle und der in der Kanüle über den Führungskatheter aufschiebbar ist, in dem ersten Zustand mit einer Innenfläche des Schirms in Eingriff steht.

Der erste und der zweite Zustand sollen dabei wahlweise einstellbar sein.

Der Begriff "konisch" ist nicht im streng geometrischen Sinne zu verstehen, sondern bezieht sich im wesentlichen auf einen von der Spitze zur Basis hin zunehmenden Durchmesser ohne sprunghafte Aufweitung, wobei die Kontur des "Konus" auch konkav oder konvex gewölbt sein kann.

Der Begriff "Basis" bezeichnet die Ebene des Schirms senkrecht zu seiner Achse, die den größten Durchmesser hat.

Der Begriff "distal" bezeichnet dabei das dem Patienten zugewandte Ende, also das Ende, das in den Patienten eingeführt wird, im Gegensatz zu "proximal", womit das dem Arzt zugewandte Ende bezeichnet wird.

Selbstverständlich ist dabei der Schirm auf dem Katheter so angeordnet, daß während des Einführens des Katheters in das Tracheostoma, mit oder ohne die Kanüle, die Spitze des Schirms in Richtung des distalen Endes des Katheters zeigt und die Basis dementsprechend proximal gelegen ist.

Ein Vorteil der erfindungsgemäßen Vorrichtung besteht darin, daß die distale Stirnfläche der Kanülenwand durch den Schirm vollständig abgedeckt wird und dadurch eine Verletzung beim Einbringen der Kanüle vermieden werden kann. Vor der Verwendung dieser Vorrichtung wird der Führungskatheter mit dem Schirm vom distalen Ende her in die Trachealkanüle eingeführt. Der Schirm kann dadurch optimal plaziert werden und muß erst nach dem Einsetzen der Trachealkanüle in die Luftröhre des Patienten durch die Kanüle hindurch zurückgezogen werden. Eine Anbringung des Schirmes ist auf jedem beliebigen Katheter, der für diesen Zweck geeignet ist, möglich. Das distale Ende des Führungskatheters sollte dabei möglichst weich und abgerundet sein, um Verletzungen vorzubeugen.

Die Änderung des Zustands des Schirms erfolgt dabei vorzugsweise durch gezielte Relativbewegung zwischen Führungskatheter und Kanüle, also beispielsweise durch Festhalten der eingesetzten Kanüle und Zurückziehen des Führungskatheters.

Selbstverständlich ist die erfindungsgemäße Vorrichtung auch beim Einsetzen anderer Tuben in künstliche oder natürliche Körperöffnungen beispielsweise beim Einbringen von Endotrachealtuben anwendbar.

Mit Hilfe der erfindungsgemäßen Vorrichtung kann nach erfolgter Vordilatation (z.B. mit einem üblichen 14 French Dilatator) die Dilatation und das Einführen der Trachealkanüle in einem Schritt erfolgen. Selbstverständlich ist es auch möglich, die Vorrichtung nach der Dilatation mit handelsüblichen Dilatatoren einzusetzen.

Die erfindungsgemäße Vorrichtung hat weiterhin den Vorteil, daß der Führungskatheter aufgrund des daran befestigten Schirms nicht vom distalen Ende in die Einführhilfe oder den Dilatator verrutschen kann. Dies vermindert zusätzlich das Risiko von Verletzungen, da in diesen Fällen die Möglichkeit bestünde, daß der ungeschützte Führungsdraht abknicken und dadurch die Einführhilfe oder der Dilatator die Rückwand der Trachea verletzen könnte.

Es ist hierzu bevorzugt, daß der äußere Basisdurchmesser des Schirms in dem ersten Zustand dem Außendurchmesser der Trachealkanüle entspricht, eine Abweichung (insbesondere Verringerung) des Basisdurchmessers gegenüber dem Außendurchmesser der Kanüle um etwa 50% der Wandstärke der Trachealkanüle ist jedoch tolerierbar, insbesondere, wenn die Stirnseite der Kanüle abgerundet ist.

In einer bevorzugten Ausführungsform hat der Schirm in dem ersten Zustand im wesentlichen die Form eines Hohlkegels und ist innen und außen konisch geformt. Die Ausformung des Schirms als Hohlkegel hat den Vorteil, daß es verschiedene Möglichkeiten der Verformung gibt, durch die der Schirm in den zweiten Zustand gebracht werden kann. Es ist möglich, daß der geringere Basisaußendurchmesser durch Eindellung der Hohlkegelwand, ähnlich dem Zusammenfalten eines Regenschirms, erreicht wird. Bevorzugt ist jedoch, daß der Schirm so ausgearbeitet ist, daß er sich von der Spitze her teilweise oder ganz umstülpt, um in den zweiten Zustand zu kommen.

Vorteilhaft ist es, wenn der Schirm eine maximale Wandstärke an der Basis von höchstens 2,5 mm, bevorzugt höchstens 1,5 mm, und wenigstens 0,4 mm, bevorzugt wenigstens 0,5 mm, aufweist. Diese Wandstärken sind dick genug, um dem Druck, der auf dem Schirm beim Einbringen der Kanüle in das Tracheostoma lastet, standzuhalten. Die Wandstärken sind aber auch dünn genug, damit der Schirm in den zweiten Zustand gebracht werden kann und dadurch problemlos durch die Trachealkanüle rückziehbar ist. Vorzugsweise verjüngt sich die Wand des Schirms von der Basis bis zur Spitze. Dadurch wird eine ausreichende Stabilisierung beim Einbringen der Trachealkanüle bei gleichzeitiger Rückziehbarkeit des Schirms durch die Kanüle erreicht.

In einer bevorzugten Ausführungsform der Erfindung befindet sich die Basis des Schirms in einem Abstand von minimal 2, besser 5 bis 14 cm, bevorzugt 7 bis 10 cm, vom distalen Ende des Führungskatheters. Beim Einführen der Kanüle wird zunächst ein Führungsdraht in das Tracheostoma eingebracht, über den daraufhin der Führungskatheter eingeführt wird. Dieser Führungskatheter dient als Führung für das anschließende Einbringen der Trachealkanüle, er muß daher ausreichend weit in die Luftröhre hineinragen. Ein zu langes überstehendes distales Ende des Führungskatheters von der Trachealkanüle könnte andererseits zu Verletzungen der Luftröhre führen.

Bevorzugt beträgt die axiale Länge des Schirms zwischen 1 cm und 6 cm, besonders bevorzugt zwischen 1,5 cm und 3 cm. Ein Schirm mit einer größeren axialen Länge wäre aus sterischen Gründen nur sehr schwer oder gar nicht durch die Kanüle rückziehbar. Um jedoch ein möglichst schonendes Einführen zu ermöglichen und das Stoma nur langsam zu dehnen, soll der Schirm eine Mindestlänge von 1 cm aufweisen.

Bevorzugt ist auch, daß der Konuswinkel zwischen dem Schirm und der Längsachse des Führungskatheters zwischen 3° und 30°, bevorzugt zwischen 5° und 15° beträgt. Schirme mit besonders kleinem Konuswinkel bewirken ein besonders schonendes Einführen in die Trachea bzw. eine langsame Dilatation des Stomas. Ein zu kleiner Winkel würde aber bei gegebenen Mindestdurchmessern von Kanülen zwangsläufig zu sehr langen Schirmen führen, die schwieriger zu handhaben wären.

Gemäß einer besonderen Ausführungsform ist vorgesehen, daß ein hohlzylinderförmiger Verdrängungskörper, dessen Außendurchmesser kleiner ist als der Innendurchmesser der Kanüle und der in der Kanüle über den Führungskatheter aufschiebbar ist, in dem ersten Zustand mit einer Innenfläche des Schirms in Eingriff steht. Dieser Verdrängungskörper dient der Stabilisierung des Schirmes. Bei Verwendung des Verdrängungskörpers kann ein größerer Druck über den Schirm auf die Trachea ausgeübt werden, ohne daß der Schirm sich nennenswert verformt oder gar umstülpt, was das Aufweiten des Stomas erleichtert. Der Verdrängungskörper ist nach dem Einbringen der Trachealkanüle unabhängig von dem Führungskatheter zurückziehbar, wodurch der Führungskatheter in der Kanüle mehr Spiel erhält und das Zurückziehen des Führungskatheters mit dem verformten Schirm vereinfacht wird.

Es besteht aber auch die Möglichkeit, den Verdrängungskörper so zu gestalten, daß er im wesentlichen aus einem kurzen Hohlzylinder besteht, dessen Durchmesser wenig geringer ist als der Innendurchmesser der Kanüle und der nach dem Einschieben im distalen Ende der Kanüle zu liegen kommt, an den ein flexibler röhrenförmiger Ansatz anschließt, der länger ist als die Kanüle und dem Einführen und Wiederherausziehen des Verdrängungskörpers dient.

Bevorzugt ist auch eine Ausführungsform, bei der der Außendurchmesser des Verdrängungskörpers mindestens 0,2 mm, bevorzugt mindestens 1 mm und höchstens 4 mm, bevorzugt höchstens 1,5 mm kleiner ist als der Innendurchmesser der Trachealkanüle. Der Verdrängungskörper wird in der Regel vom proximalen Ende der Trachealkanüle eingeschoben. Ist sein Außendurchmesser kleiner als der Innendurchmesser der Trachealkanüle, so wird ein Einschieben auch bei Kanülen mit sehr flexiblen Wänden oder sehr kleinem Innendurchmesser erleichtert. Ist der Durchmesser des Verdrängungskörpers zu klein, so kann er den Schirm beim Einführen der Trachealkanüle nicht mehr ausreichend unterstützen.

Besonders bevorzugt ist eine Ausführungsform, bei welcher die Basis des Schirms einen hohlzylinderförmigen Ansatz aufweist, mit einem Außendurchmesser, der dem Innendurchmesser der Kanüle angepaßt ist und diesen um maximal 10%, bevorzugt maximal 5%, bezogen auf den Innendurchmesser, über- oder unterschreitet, und mit einer Wandstärke, die zwischen 0,5 mm und 2 mm, bevorzugt zwischen 0,5 mm und 1 mm, beträgt. Durch diesen Ansatz entsteht eine Stufe zwischen der Basis der konischen Spitze und dem hohlzylinderförmigen Ansatz des Schirmes. Die radiale Tiefe der Stufe entspricht im ersten Zustand im wesentlichen der Dicke des Kanülenrohrs. Beim Einschieben des Führungskatheters mit dem Schirm vom distalen Ende in die Kanüle wird der Ansatz in das Kanülenrohr geschoben, während die Basis des Schirms auf der distalen Stirnfläche des Kanülenrohrs zu liegen kommt. Diese Ausführungsform bedingt eine verbesserte Stabilisierung des Schirms, so daß dieser bei Druck in Richtung des Kanülenrohrs nicht versehentlich umklappt oder zusammengestaucht wird. Auch ein Verschieben der Einführhilfe relativ zur Längsachse der Kanüle wird dadurch verhindert, wodurch dem Arzt ein kontrollierteres Einführen oder Dilatieren ermöglicht wird.

Bei Verwendung eines Verdrängungskörpers ist es günstig, wenn der zylinderförmige Ansatz an der Basis des Schirms einen Außendurchmesser hat, der dem Innendurchmesser der Kanüle angepaßt ist und diesen um maximal 10%, bevorzugt maximal 5%, bezogen auf den Innendurchmesser über- oder unterschreitet, und einen Innendurchmesser, der im wesentlichen dem Außendurchmesser des Verdrängungskörpers entspricht oder etwas kleiner ist, aufweist. Dadurch befindet sich der hohlzylinderförmige Ansatz in dem ersten Zustand des Schirms zwischen dem Kanülenrohr und dem Verdrängungskörper. Zum Aufschieben des Schirms auf den Verdrängungskörper ist es dabei von Vorteil, wenn der hohlzylinderförmige Ansatz sich in proximaler Richtung verjüngt.

Bevorzugt ist weiterhin eine Vorrichtung, bei der die axiale Länge des hohlzylinderförmigen Ansatzes an der Basis des Schirmes zwischen 0,3 und 5 mm, bevorzugt zwischen 1 und 3 mm, beträgt. Für eine optimale Stabilisierung zur Verhinderung des Zusammenklappens des Schirms während des Eindringens der Kanüle muß der hohlzylinderförmige Ansatz ausreichend lang sein. Ein zu langer Ansatz könnte hingegen bei der Verformung des Schirmes hinderlich sein oder diese womöglich gänzlich verhindern.

Bevorzugt ist auch eine Ausführungsform, bei der der Verdrängungskörper an seinem distalen Ende eine konische Spitze aufweist. Zweckmäßig ist es, wenn diese Spitze komplementär zu einer konischen Innenfläche des Schirmes ausgebildet ist. Dadurch wird der Schirm beim Einführen der Kanüle besonders gut stabilisiert.

Besonders bevorzugt besteht der Schirm aus einem im wesentlichen elastischen Material. Wird ein solcher elastischer Schirm zusammen mit einem Verdrängungskörper verwendet, so sollte der Schirm einen Innendurchmesser aufweisen, der den Außendurchmesser des Verdrängungskörpers um 0-40%, bevorzugt 20-30%, bezogen auf den Außendurchmesser des Verdrängungskörpers unterschreitet. Dadurch besteht die Möglichkeit, daß der Schirm über die Spitze des Verdrängungskörpers gespannt werden kann, was die Stabilität und den Halt des Schirmes und des Verdrängungskörpers erhöht, insbesondere wenn ein Schirm mit einem hohlzylinderförmigen Ansatz an der Basis verwendet wird. Bei der Verwendung eine elastischen Schirms in der erfindungsgemäßen Vorrichtung kann der zweite Zustand des Schirms dem ungespannten Zustand des Schirms entsprechen. Ein solcher Schirm ist ohne Umstülpen durch die Trachealkanüle rückziehbar.

Weiterhin ist eine Ausgestaltung der Erfindung bevorzugt, bei welcher der Schirm und/oder der Verdrängungskörper einen im wesentlichen ovalen Querschnitt aufweist. Der Schirm und/oder der Verdrängungskörper sollten dabei so ausgerichtet werden, daß ihre Ausdehnung senkrecht zu den Trachealspangen des Patienten kleiner ist, als in Richtung des Verlaufs der Trachealspangen. Dies kann beispielsweise allein oder überwiegend durch eine senkrecht zu den Trachealspangen verringerte bzw. parallel zu den Trachealspangen vergrößerte Wandstärke erreicht werden, wobei die Differenz der Wandstärken beispielsweise zwischen 0,25 mm und 1 mm betragen kann. Da ein Aufdehnen des Stomas senkrecht zu den Trachealspangen schwieriger ist, als zwischen den Spangen, kann diese Ausführungsform leichter in das Stoma eingeführt werden oder eine einfachere Dilatation des Stomas bewirken. Ein weiterer Vorteil dieser Ausführungsform besteht darin, daß die Trachealkanüle, die in der Regel einen runden Querschnitt aufweist, durch einen ovalen Schirm und/oder ovalen Verdrängungskörper in Richtung der Trachealspangen aufgeweitet und/oder senkrecht dazu reduziert werden kann. Diese ovale Deformation erleichtert das Einführen der Kanüle in die Trachea wie oben erläutert. Dies wird insbesondere dann erreicht, wenn der Verdrängungskörper einen ovalen Querschnitt aufweist. Wird der Verdrängungskörper entfernt, so nimmt die Trachealkanüle wieder ihre ursprüngliche runde Querschnittsform an. Die gleiche Wirkung kann durch einen Verdrängungskörper erzielt werden, der wenigstens am distalen Ende eine ovale Form aufweist.

Besonders bevorzugt ist eine Ausführungsform, bei der der Verdrängungskörper eine herkömmliche Einführhilfe ist. Die aus dem Stand der Technik bekannten Einführhilfen, bei denen zwischen Einführhilfe und distalem Ende der Kanüle eine Stufe auftritt, stellen eine spezielle Ausführung eines Verdrängungskörpers dar. Durch die Verwendung solcher herkömmlicher Einführhilfen verringern sich die Kosten der Herstellung der erfindungsgemäßen Vorrichtung, da bereits auf dem Markt befindliche Kanülen mit Einführhilfe verwendet werden können und lediglich ein Führungskatheter mit dem erfindungsgemäßen Schirm den herkömmlichen Katheter ersetzen muß.

Vorzugsweise besteht der Schirm aus einem elastischen Material wie Silikon, einem thermoplastischen Elastomer (TPE), Latex oder Polyurethan. Diese Materialien sind geeignet, um einen ausreichend flexiblen und gegebenenfalls elastischen Schirm herzustellen, der gleichzeitig über die notwendige Stabilität verfügt. Im übrigen können diese Materialien entsprechend den Anforderungen im medizinischen Bereich behandelt werden, so daß sie steril sind.

Bevorzugt ist auch eine Ausführungsform, bei der der Schirm mit einer hydrophilen Beschichtung versehen ist. Eine solche Beschichtung hat vorzugsweise nach Befeuchtung einen geringen Reibungskoeffizienten und erleichtert dadurch das Einführen dar Kanüle. Selbstverständlich ist es ausreichend, wenn die Außenfläche des Schirms mit einer solchen Beschichtung überzogen ist. Alternativ dazu kann der Schirm auch auf seiner Außenfläche mit Gleitgel benetzt sein, dies hat die gleiche Funktion wie die oben beschriebene Beschichtung.

Weiterhin bevorzugt ist eine Ausführungsform, bei der die Spitze des Schirmes einen hohlzylinderförmigen Ansatz aufweist. Dieser Ansatz dient im wesentlichen der Befestigung des Schirmes an dem Führungskatheter. Er kann seinerseits über eine konische Spitze verfügen, so daß zwischen dem Führungskatheter und diesem distalen Ansatz kein Kalibersprung vorhanden ist. Besonders vorteilhaft ist es, wenn die konische Spitze einer Verlängerung des Schirms darstellt und die Außenflächen der Spitze und des Schirms dadurch eine durchgehende Fläche ohne ausgeprägten Übergang bilden.

Besonders bevorzugt ist eine Ausführungsform, bei welcher der Schirm durch Verkleben oder Verschweißen mit dem Führungskatheter verbunden ist. Diese Art der Befestigung ist stark genug, um ein Ablösen des Schirmes vom Führungskatheter beispielsweise während des Zurückziehens zu verhindern. Der Führungskatheter könnte auch - beispielsweise durch Spritzgießen - einstückig mit dem Schirm hergestellt werden.

Weitere Vorteile, Merkmale und Anwendungsmöglichkeiten der vorliegenden Erfindung werden deutlich anhand der folgenden Beschreibung bevorzugter Ausführungsformen und den dazu gehörigen Figuren. Es zeigen:
- Figur 1: einen Führungskatheter mit Schirm in einer ersten Ausführungsform,
- Figur 2: einen Führungskatheter mit einem Schirm in einer weiteren Ausführungsform,
- Figur 3: einen Längsschnitt durch eine Trachealkanüle mit Führungskatheter, Verdrängungskörper und Schirm in einem ersten Zustand,
- Figur 4: einen Längsschnitt durch eine Trachealkanüle mit Führungskatheter, Verdrängungskörper und Schirm, wobei der Verdrängungskörper zurückgezogen wird,
- Figur 5: einen Längsschnitt durch eine Trachealkanüle mit Führungskatheter und Schirm beim Übergang von dem ersten zu dem zweiten Zustand,
- Figur 6: einen Längsschnitt durch eine Trachealkanüle mit Führungskatheter und Schirm in dem zweiten Zustand,
- Figur 7: eine Trachealkanüle, in die ein Führungskatheter mit Schirm eingeschoben wird, und
- Figur 8: einen Führungskatheter mit Schirm in einer weiteren Ausführungsform

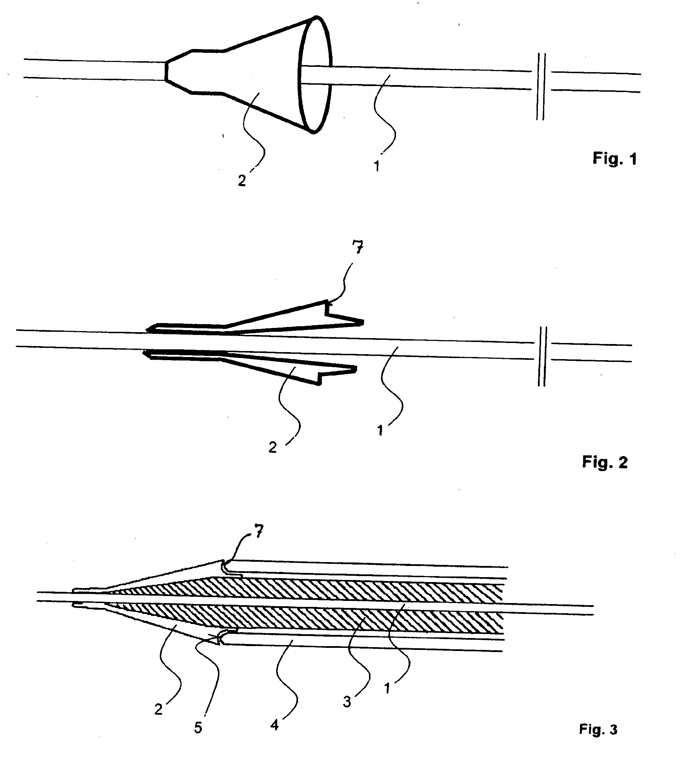

In Figur 1 ist ein Führungskatheter 1 mit einer ersten Ausführungsform eines Schirmes 2 dargestellt. Der Schirm hat die Form eines Hohlkegels mit im wesentlichen konischen Innen- und Außenflächen. Am distalen Ende des Schirmes 2 ist ein hohlzylinderförmiger Ansatz gezeigt, der seinerseits über eine im wesentlichen konische Spitze verfügt, die einen Kalibersprung zwischen dem Führungskatheter 1 und dem Schirm 2 verhindert. Der Schirm 2 befindet sich auf dem Führungskatheter 1 auf Höhe des Sicherheitsstoppers bzw. anstelle des Sicherheitsstoppers, der bei den bekannten Einführhilfen dafür sorgt, daß die Einführhilfe nicht versehentlich komplett über den Führungskatheter 1 geschoben werden kann.

Bei dem Sicherheitsstopper herkömmlicher Führungskatheter handelt es sich um eine Verdickung des Katheterrohres. Wird der Schirm bei der erfindungsgemäßen Vorrichtung so angebracht, daß sich eine solche Verdickung unmittelbar proximal der Spitze des Schirmes befindet, so wird dadurch die Spitze des Schirmes besonders stabilisiert und auch die Befestigung des Schirmes erleichtert, ohne die Rückziehbarkeit des Katheters mit dem Schirm durch die Trachealkanüle einzuschränken.

Figur 2 stellt einen Längsschnitt durch eine weitere Ausführungsform eines Führungskatheters 1 mit Schirm 2 dar. Der Schirm 2 verfügt an seinem distalen Ende über einen dünnwandigen hohlzylinderförmigen Ansatz, welcher zugespitzt ist. Der Ansatz stellt eine langgezogene Spitze des konischen Schirmes 2 dar und bewirkt, daß zwischen Schirm 2 und Führungskatheter 1 eine ausreichende Fläche vorhanden ist, um diese durch Verkleben oder Verschweißen miteinander zu verbinden. Weiterhin verfügt der hier dargestellte Schirm an seiner Basis über einen hohlzylinderförmigen Ansatz, dessen Außendurchmesser kleiner ist als der Durchmesser der Basis des Schirms und dessen Innendurchmesser größer ist als der Außendurchmesser des Verdrängungskörpers. Die Innenfläche des Ansatzes stellt eine Fortsetzung der Innenfläche des Schirms 2 dar und der Ansatz verjüngt sich in proximaler Richtung.

In Figur 3 ist ein Längsschnitt des distalen Endes einer Trachealkanüle 4, einem Führungskatheter 1 mit einer weiteren Ausführungsform eines Schirmes 2 und einem Verdrängungskörper 3 dargestellt. Der Ansatz an der Basis des Schirms 2 befindet sich zwischen dem Kanülenrohr 4 und dem Verdrängungskörper 3. Der Verdrängungskörper 3 fixiert so den Schirm 2 in einer Position, in der der Schirm 2 den Kalibersprung zwischen dem Führungskatheter 1 und dem Verdrängungskörper 3 mit dem Kanülenrohr 4 ausgleicht. Dadurch wird auch verhindert, daß beim Einführen der Kanüle 4 mit Hilfe der erfindungsgemäßen Vorrichtung der Schirm 2 zu früh umklappt oder sich deformiert. Die Basis des Schirms 2 deckt die distale Stirnfläche 5 des Kanülenrohres 4 vollständig ab und verhindert dadurch Verletzungen, die beim Hängenbleiben dieser Fläche an der Trachea entstehen können. Der Längsschnitt zeigt deutlich, daß der Verdrängungskörper 3 über ein zentrales Lumen für die Aufnahme des Führungskatheters 1 verfügen muß. Es ist sonst nicht möglich, daß der Führungskatheter 1 mit Schirm 2 und der Verdrängungskörper 3 sich gleichzeitig innerhalb der Kanüle 4 befinden. Der Verdrängungskörper könnte auch allein aus einer konischen Spitze, gegebenen falls mit einem kurzen hohlzylindrischen Ansatz bestehen, der über einen Schlauch oder ein oder mehrere Zugdrähte oder -fäden mit der proximalen Seite dieser Spitze verbunden ist, und auf diese Weise aus der in Figur 3 dargestellten Position zurückgezogen werden kann.

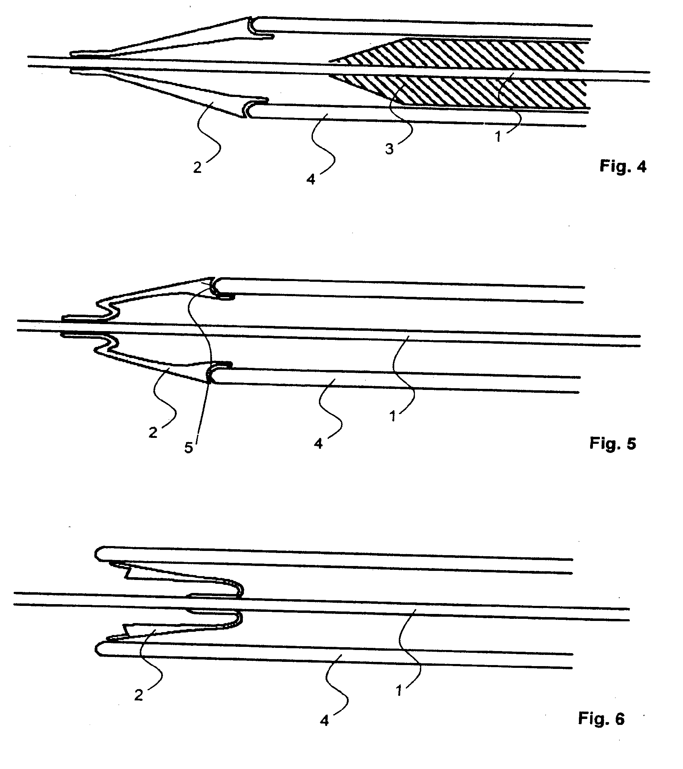

Figur 4 zeigt das distale Ende einer Kanüle 4 mit Verdrängungskörper 3 und dem Führungskatheter 1 mit Schirm 2 aus Figur 3. Dabei ist der Verdrängungskörper 3 leicht in Richtung proximales Ende der Kanüle 4 verschoben, so daß die Fixierung des Schirms 2 sowie die Fixierung des Verdrängungskörpers 3 selbst innerhalb der Kanüle 4 aufgehoben ist. Durch Zurückziehen des Führungskatheters 1 in Richtung proximales Ende der Kanüle 4 könnte die Basis des Schirms, je nach Ausgestaltung der Stirnfläche der Kanüle und der dieser anliegenden Basisfläche des Schirms, sich einwärts falten und in die Kanüle hineingleiten.

In Figur 5 ist die Kanüle 4 mit dem Führungskatheter 1 und dem Schirm 2 aus Figur 3 gezeigt. Der Verdrängungskörper 3 wurde dabei bereits aus der Trachealkanüle entfernt. Durch leichtes Rückziehen des Führungskatheters 1 in proximale Richtung wird der Schirm 2 von dem ersten Zustand, in welchem er den Kalibersprung zwischen Kanüle und Verdrängungskörper überbrückt, von der Spitze her einwärts in den zweiten Zustand umgestülpt, in welchem der Führungskatheter 1 mit dem Schirm 2 durch die Kanüle 4 rückziehbar ist, wobei er zunächst noch die distale Stirnfläche 5 der Kanüle 4 abdeckt.

Figur 6 zeigt die Kanüle 4 mit dem Führungskatheter 1 und dem Schirm 2 aus Figur 3, wobei sich der Schirm endgültig in dem zweiten Zustand befindet. Durch Zurückziehen des Führungskatheters 1 in Richtung proximales Ende der Kanüle 4 wurde der Schirm 2 so weit verformt, bis er vollständig umgestülpt wurde. Dabei kann der Schirm in seinem Basisbereich auch durchaus Falten werfen (nicht dargestellt) In diesem umgestülpten Zustand kann der Schirm 2 durch die Kanüle 4 in Richtung proximales Ende zurückgezogen werden.

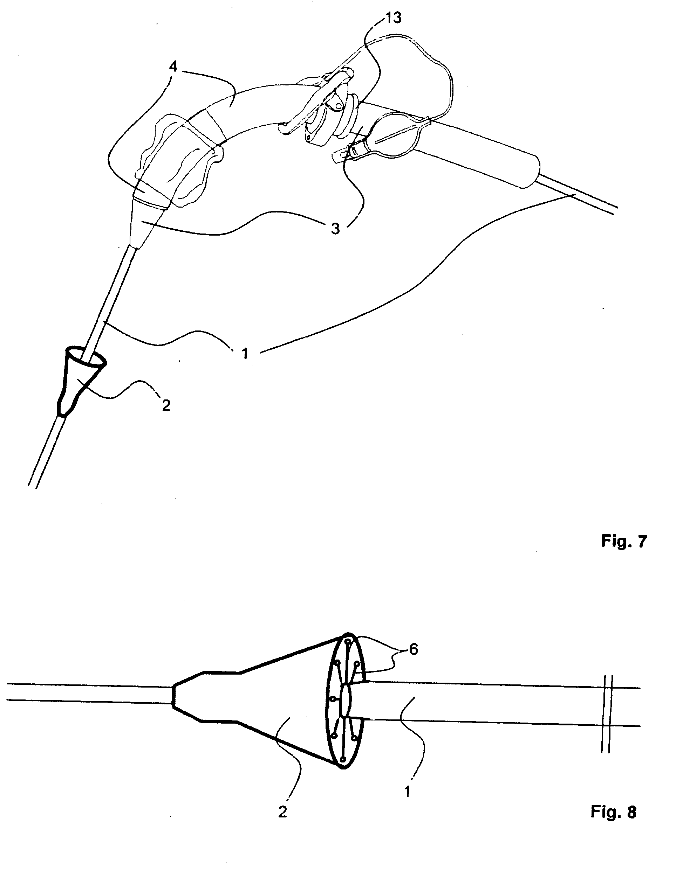

In Figur 7 ist gezeigt, wie ein Führungskatheter 1 mit Schirm 2 in einer Ausführung wie in Figur 1 dargestellt, vom distalen Ende her in eine Kanüle 4, in der sich eine Einführhilfe 3 als Verdrängungskörper befindet, eingeschoben wird. Bei diesem Verdrängungskörper handelt es sich um eine gängige Einführhilfe 3 mit einem ringförmigen Ansatz 13, der verhindert, daß die Einführhilfe zu weit in Richtung distales Ende der Kanüle 4 geschoben wird.

Figur 8 stellt eine Ausführungsform eines Schirmes 2 dar. Bei dieser Ausführungsform zeichnet sich der Schirm durch eine besonders große Wanddicke aus, die zwischen 1,5 und 4 mm liegen kann. Die Einschnitte 6, deren Tiefe bis zu 90% der Wandstärke betragen kann, ermöglichen ein Umstülpen des Schirms. Diese Einschnitte enden jeweils in einer Aufweitung mit einem im wesentlichen kreisförmigem Querschnitt, was ein Aufreißen des Schirms beim Umstülpen verhindert. Die Basis des Schirms geht dabei von einer ebenen Fläche in eine gefurchte, gewölbte Form über. Weiterhin kann der Schirm in dieser Ausführungsform aus einem komprimierbaren Material bestehen, damit der Führungskatheter 1 gemeinsam mit diesem Schirm 2 durch eine Trachealkanüle leichter rückziehbar ist. Der Führungskatheter 1 ist bei dieser Ausführungsform des Schirms so gestaltet, daß er proximal des Schirmes einen größeren Außendurchmesser hat. Der Katheter übernimmt somit die Funktion eines Verdrängungskörpers und trägt damit zur Stabilisierung des Schirmes beim Einführen der Trachealkanüle in die Trachea des Patienten bei. Im umgestülpten Zustand umschließt die Basis des Schirmes einen Katheterabschnitt mit kleinerem Außendurchmesser und ist dadurch ausreichend komprimierbar oder verformbar, um durch die Kanüle zurückgezogen zu werden.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

## Patentansprüche

1. Vorrichtung mit einem Führungskatheter (1) zum Einbringen einer Trachealkanüle (4) in ein Tracheostoma, wobei der Führungskatheter (1) einen Schirm (2) aufweist, der in der Nähe des distalen Endes des Führungskatheters (1) angebracht ist und der aus einem flexiblen Material besteht und im wesentlichen die Form einer distal gerichteten konischen Spitze hat, wobei das Rohr des Führungskatheters (1) durch die Achse dieser Spitze verläuft, mit einem Basisaußendurchmesser, der in einem ersten Zustand in etwa dem Außendurchmesser der Trachealkanüle (4) entspricht und in einem zweiten Zustand durch Verformung kleiner ist als der Innendurchmesser der Trachealkanüle (4) und damit durch die Trachealkanüle rückziehbar ist, weiterhin enthaltend einen hohlzylinderförmigen Verdrängungskörper (3), dessen Außendurchmesser kleiner ist als der Innendurchmesser der Kanüle (4) und der in der Kanüle über den Führungskatheter (1) aufschiebbar ist, welcher in dem ersten Zustand mit einer Innenfläche des Schirms (2) in Eingriff steht.

2. Vorrichtung nach Anspruch 1, **dadurch gekennzeichnet, daß** der Schirm (2) in dem ersten Zustand im wesentlichen die Form eines Hohlkegels hat und innen und außen konisch ist.

3. Vorrichtung nach Anspruch 1 oder 2, **dadurch gekennzeichnet, daß** der Schirm (2) eine maximale Wandstärke an der Basis von höchstens 2,5 mm, bevorzugt höchstens 1,5 mm, und wenigstens 0,4 mm, bevorzugt wenigstens 0,5 mm, aufweist.

4. Vorrichtung nach einem der vorangehenden Ansprüche, **dadurch gekennzeichnet, daß** sich die Basis des Schirms (2) in einem Abstand von mindestens 2, besser mindestens 5 bis 14 cm, besonders bevorzugt 7 bis 10 cm vom distalen Ende des Führungskatheters (1) befindet.

5. Vorrichtung nach einem der vorangehenden Ansprüche, **dadurch gekennzeichnet, daß** der Schirm (2) eine axiale Länge von wenigstens 1 cm, bevorzugt wenigstens 1,5 cm, und höchstens 6 cm, bevorzugt höchstens 3 cm, aufweist.

6. Vorrichtung nach einem der vorangehenden Ansprüche, **dadurch gekennzeichnet, daß** der Konuswinkel zwischen dem Schirm (2) und der Längsachse des Führungskatheters (1) zwischen 3° und 30°, bevorzugt zwischen 5° und 15° beträgt.

7. Vorrichtung nach einem der vorangehenden Ansprüche, **dadurch gekennzeichnet, daß** der Außendurchmesser des Verdrängungskörpers (3) mindestens 0,2 mm, bevorzugt mindestens 1 mm und höchstens 4 mm, bevorzugt höchstens 1,5 mm kleiner ist als der Innendurchmesser der Trachealkanüle (4).

8. Vorrichtung nach einem der vorangehenden Ansprüche, **dadurch gekennzeichnet, daß** die Basis des Schirms (2) einen hohlzylinderförmigen Ansatz aufweist, mit einem Außendurchmesser, der dem Innendurchmesser der Kanüle (4) angepaßt ist und diesen um maximal 10%, bevorzugt um maximal 5%, bezogen auf den Innendurchmesser über- oder unterschreitet, und mit einer Wandstärke, die zwischen 0,5 mm und 2 mm, bevorzugt zwischen 0,5 und 1 mm, beträgt.

9. Vorrichtung nach Anspruch 8, **dadurch gekennzeichnet, daß** die axiale Länge des hohlzylinderförmigen Ansatzes zwischen 0,3 und 5 mm, bevorzugt zwischen 1 und 3 mm, beträgt.

10. Vorrichtung nach einem der vorangehenden Ansprüche, **dadurch gekennzeichnet, daß** der Verdrängungskörper (3) an seinem distalen Ende eine konische Spitze aufweist.

11. Vorrichtung nach einem der vorangehenden Ansprüche, **dadurch gekennzeichnet, daß** der Schirm (2) aus einem im wesentlichen elastischen Material besteht.

12. Vorrichtung nach einem der vorangehenden Ansprüche, **dadurch gekennzeichnet, daß** die Spitze des Schirms (2) distal einen hohlzylinderförmigen Ansatz aufweist.

13. Vorrichtung nach einem der vorangehenden Ansprüche, **dadurch gekennzeichnet, daß** der Schirm (2) durch Verkleben oder einstückig durch Spritzguß oder Verschweißen mit dem Führungskatheter (1) verbunden ist.

## Claims

1. A device having a guide catheter (1) for the introduction of a tracheal cannula (4) into a tracheostoma, wherein the guide catheter (1) has a shield (2) which is mounted in the proximity of the distal end of the guide catheter (1) and which comprises a flexible material and which is substantially in the form of a distally directed conical tip, wherein the tube of the guide catheter (1) passes through the axis of said tip, with a base outside diameter which in a first state corresponds approximately to the outside diameter of the tracheal cannula (4) and in a second state by deformation is smaller than the inside diameter of the tracheal cannula (4) and is thus retractable through the tracheal cannula, further comprising a displacement body (3) in the form of a hollow cylinder, the outside diameter of which is smaller than the inside diameter of the cannula (4) and which can be pushed on over the guide catheter (1) in the cannula and which is in engagement with an inside surface of the shield (2) in the first state.

2. A device according to claim 1 **characterised in that** the shield (2) in the first state is substantially in the form of a hollow cone and is internally and externally conical.

3. A device according to claim 1 or claim 2 **characterised in that** the shield (2) has a maximum wall thickness at the base of at most 2.5 mm, preferably at most 1.5 mm, and at least 0.4 mm, preferably at least 0.5 mm.

4. A device according to one of the preceding claims **characterised in that** the base of the shield (2) is at a spacing of at least 2, better at least 5 to 14 cm, particularly preferably 7 to 10 cm from the distal end of the guide catheter (1).

5. A device according to one of the preceding claims **characterised in that** the shield (2) is of an axial length of at least 1 cm, preferably at least 1.5 cm and at most 6 cm, preferably at most 3 cm.

6. A device according to one of the preceding claims **characterised in that** the cone angle between the shield (2) and the longitudinal axis of the guide catheter (1) is between 3° and 30°, preferably between 5° and 15°.

7. A device according to one of the preceding claims **characterised in that** the outside diameter of the displacement body (3) is at least 0.2 mm, preferably at least 1 mm and at most 4 mm, preferably at most 1.5 mm smaller than the inside diameter of the tracheal cannula (4).

8. A device according to one of the preceding claims **characterised in that** the base of the shield (2) has an attachment in the form of a hollow cylinder, of an outside diameter which is adapted to the inside diameter of the cannula (4) and which is larger than or smaller than same by a maximum of 10%, preferably by a maximum of 5%, with respect to the inside diameter, and of a wall thickness which is between 0.5 mm and 2 mm, preferably between 0.5 and 1 mm.

9. A device according to claim 8 **characterised in that** the axial length of the attachment in the form of a hollow cylinder is between 0.3 and 5 mm, preferably between 1 and 3 mm.

10. A device according to one of the preceding claims **characterised in that** the displacement body (3) has a conical tip at its distal end.

11. A device according to one of the preceding claims **characterised in that** the shield (2) consists of a substantially elastic material.

12. A device according to one of the preceding claims **characterised in that** the tip of the shield (2) distally has an attachment in the form of a hollow cylinder.

13. A device according to one of the preceding claims **characterised in that** the shield (2) is joined to the guide catheter (1) by adhesive or integrally by injection moulding or welding.

## Revendications

1. Dispositif pourvu d'un cathéter-guide (1) destiné à l'insertion d'une canule trachéale (4) dans un trachéostome, dans lequel le cathéter guide (1) présente un parapluie (2) qui est appliqué à proximité de l'extrémité distale du cathéter-guide (1), se compose d'un matériau flexible et a sensiblement la forme d'une pointe conique d'orientation distale, le tube du cathéter-guide (1) s'étendant à travers l'axe de cette pointe, et présentant un diamètre extérieur de base qui dans un premier état correspond sensiblement au diamètre extérieur de la canule trachéale (4) et dans un second état, par déformation, est plus petit que le diamètre intérieur de la canule trachéale (4) et peut par conséquent être rétracté à travers la canule trachéale, comprenant en outre un corps de déplacement (3) en forme de cylindre creux dont le diamètre extérieur est plus petit que le diamètre intérieur de la canule (4) et qui peut être enfilé dans la canule (4) sur le cathéter guide (1), lequel, dans le premier état, est en prise avec une surface intérieure du parapluie (2).

2. Dispositif selon la revendication 1, **caractérisé en ce que** le parapluie (2) dans le premier état a sensiblement la forme d'un cône creux et est conique à l'intérieur et à l'extérieur.

3. Dispositif selon la revendication 1 ou 2, **caractérisé en ce que** le parapluie (2) présente une épaisseur de paroi maximale au niveau de la base de 2,5 mm maximum, de préférence 1,5 mm maximum, et d'au moins 0,4 mm, de préférence au moins 0,5 mm.

4. Dispositif selon l'une des revendications précédentes, **caractérisé en ce que** la base du parapluie (2) se trouve à une distance d'au moins 2, préférablement d'au moins 5 à 14 cm, de façon particulièrement préférée de 7 à 10 cm, de l'extrémité distale du cathéter-guide (1).

5. Dispositif selon l'une des revendications précédentes, **caractérisé en ce que** le parapluie (2) présente une longueur axiale d'au moins 1 cm, de préférence d'au moins 1,5 cm, et au maximum de 6 cm, de préférence au maximum de 3 cm.

6. Dispositif selon l'une des revendications précédentes, **caractérisé en ce que** l'angle de conicité entre le parapluie (2) et l'axe longitudinal du cathéter guide (1) se situe entre 3° et 30°, de préférence entre 5° et 15°.

7. Dispositif selon l'une des revendications précédentes, **caractérisé en ce que** le diamètre extérieur du corps de déplacement (3) est plus petit que le diamètre intérieur de la canule trachéale (4) d'au moins 0,2 mm, de préférence d'au moins 1 mm et au maximum de 4 mm, de préférence au maximum de 1,5 mm.

8. Dispositif selon l'une des revendications précédentes, **caractérisé en ce que** la base du parapluie (2) présente un prolongement en forme de cylindre creux dont le diamètre extérieur est adapté au diamètre intérieur de la canule (4) et dépasse ou est inférieur à celui-ci de 10 % maximum, de préférence de 5 % maximum, par rapport au diamètre intérieur, et dont l'épaisseur de paroi est comprise entre 0,5 mm et 2 mm, de préférence entre 0,5 et 1 mm.

9. Dispositif selon la revendication 8, **caractérisé en ce que** la longueur axiale du prolongement en forme de cylindre creux est comprise entre 0,3 et 5 mm, de préférence entre 1 et 3 mm.

10. Dispositif selon l'une des revendications précédentes, **caractérisé en ce que** le corps de déplacement (3) présente une pointe conique à son extrémité distale.

11. Dispositif selon l'une des revendications précédentes, **caractérisé en ce que** le parapluie (2) se compose d'un matériau sensiblement élastique.

12. Dispositif selon l'une des revendications précédentes, **caractérisé en ce que** la pointe du parapluie (2) présente côté distal un prolongement en forme de cylindre creux.

13. Dispositif selon l'une des revendications précédentes, **caractérisé en ce que** le parapluie (2) est relié au cathéter-guide (1) par collage ou d'un seul tenant par moulage par injection ou par soudage.
